# EUROPEAN PATENT APPLICATION

(11) **EP 4 002 265 A1**
(43) Date of publication of application: **25.05.2022**
(21) Application number: 21207194.8
(22) Date of filing: 09.11.2021
(51) Int. Cl.: G06T 5/00

(54) **ELECTRONIC DEVICE CONFIGURED TO PROCESS IMAGE DATA FOR TRAINING ARTIFICIAL INTELLIGENCE SYSTEM**

(30) Priority: 16.11.2020 KR 20200152760
(71) Applicant: Bonewise Inc., Seoul 06164 (KR)
(72) Inventor: JIN, Dong Kyu, 06293 SEOUL (KR)
(74) Representative: Cabinet Beau de Loménie

(57) **Abstract**

The present disclosure includes a reception circuit (1400) and a processor (1100). The reception circuit (1400) receives input image data comprised of pixels. The processor (1100) is configured to perform at least one of a first operation of adjusting pixel values of first object pixels selected from the pixels on the basis of noise data, a second operation of adjusting pixel values of second object pixels selected on the basis of the number of pixels, a third operation of generalizing the input image data on the basis of coordinate values of inflection pixels determined on the basis of gradients between coordinate values of the pixels, and a fourth operation of obtaining input image data having pixel values in a second range by adjusting pixel values of input image data having pixel values in the first range.

## Description

### TECHNICAL FIELD

The present disclosure relates to an electronic device, and more particularly, to an electronic device configured to process image data for training an artificial intelligence system.

### BACKGROUND

Research on assessment of the bone age of a patient based on a medical image such as an X-ray image of the patient's body is underway. When the bone age of a patient is accurately assessed, the assessed bone age may be used for a variety of medical purposes. For example, the Greulich-Pyle (G&P) method or the Tanner-Whitehouse (TW) method may be used to assess the bone age of a patient.

Meanwhile, artificial intelligence technologies such as machine learning are being utilized to analyze image data representing images. As an example of machine learning, various techniques of deep learning using an artificial neural network are being studied. The artificial neural network for implementing deep learning may be trained on the basis of a large amount of data. The higher the quality of the data used in training, the higher the performance of the artificial neural network that can be obtained. The data to be used in training may be preprocessed to obtain high quality data for training.

In the medical field, deep learning technology is utilized to analyze medical images and diagnose patients. For example, the bone age of a patient may be assessed by classifying X-ray images by the artificial neural network, and a clinician may diagnose the patient on the basis of the assessed bone age. Therefore, in order to obtain a high-performance artificial neural network to be used in diagnosing patients, research on a method for processing image data to be used for training the artificial neural network is required.

### SUMMARY

The present disclosure can provide an electronic device configured to preprocess image data to be used for training an artificial intelligence system.

An electronic device according to an embodiment of the present disclosure may include a reception circuit and a processor. The reception circuit may receive input image data. The processor may be configured to perform at least one of a first operation of adjusting pixel values of first object pixels representing image data corresponding to noise data, among input pixels of the input image data, a second operation of determining sequentially adjacent line pixels among the input pixels on the basis of the pixel values of the input pixels and adjusting pixel values of second object pixels determined from among the input pixels on the basis of the number of line pixels, a third operation of adjusting coordinate values of the input pixels on the basis of coordinate values of inflection pixels determined on the basis of rates of change in the coordinate values between the line pixels, a fourth operation of adjusting pixel values of the input pixels such that the input pixels having pixel values within a first range have pixel values within a second range, a magnitude of the second range being greater than a magnitude of the first range.

Optional aspects are defined in the dependent claims.

An electronic device according to an embodiment of the present disclosure may include a reception circuit and a processor. The reception circuit may receive input image data. The processor may be configured to perform a first operation of extracting object image data from the input image data on the basis of inflection pixels included in a first pixel line of the input image data, a second operation of adjusting pixel values of object pixels determined among pixels of the input image data on the basis of a comparison between the number of pixels included in a second pixel line of the object image data and the number of pixels included in a third pixel line of the object image data, and a third operation of scaling pixel values of the object image data.

The processor may be configured to further perform a fourth operation of adjusting pixel values of image data matching noise data, among the object image data.

Each of differences between pixel values of the first pixel line and pixel values of pixels adjacent to the first pixel line may be equal to or greater than a threshold value.

The processor may be further configured to call a function for determining an array of a fourth pixel line of the object image data, and extract region image data included in the object image data based on the called function.

The processor may be further configured to determine the inflection pixels based on differences between coordinate values of pixels included in the first pixel line.

The processor may be further configured to determine the inflection pixels further based on differences between gradients calculated based on the differences between the coordinate values.

The processor may be further configured to perform a fifth operation of indexing the inflection pixels, determining reference pixels among the indexed inflection pixels based on the coordinate values of the indexed inflection pixels, and adjusting coordinate values of the object image data based on the determined coordinate values of the reference pixels.

The processor may be further configured to perform the fifth operation of inverting coordinate values of the input image data based on a comparison between the coordinate values of the reference pixels.

The processor may be further configured to extract the object image data included in the input image data based on a difference between coordinate values of the inflection pixels and a rate of change between coordinate values of the inflection pixels.

An electronic device according to an embodiment of the present disclosure may include a reception circuit and a processor. The reception circuit may receive first image data. The processor may be configured to obtain second image data by adjusting pixel values of a region included in the first image data and matching noise data, obtain third image data by adjusting pixel values of sub-image data divided from the second image data, and if a coordinate value of a first reference pixel among the pixels of the third image data is greater than a coordinate value of a second reference pixel among the pixels of the third image data, obtain fourth image data by adjusting coordinate values of the pixels of the third image data. Regions corresponding to the sub-image data may not overlap each other, and a magnitude of the range of pixel values representing the third image data may be greater than a magnitude of the range of pixel values representing the second image data.

### BRIEF DESCRIPTION OF DRAWINGS

The accompanying drawings, which are incorporated in and constitute a part of the specification, illustrate embodiments of the present disclosure.
FIG. 1 is a block diagram illustrating an electronic device configured to process data according to an embodiment of the present disclosure.
FIG. 2 is a flowchart illustrating exemplary operations of an electronic device for processing image data.
FIG. 3 is a flowchart illustrating exemplary operations of an electronic device for processing image data.
FIG. 4 is a conceptual diagram illustrating an embodiment of operation S111.
FIG. 5 is a conceptual diagram illustrating exemplary image data configured in units of pixels.
FIG. 6 is a conceptual diagram illustrating exemplary boundary pixels constituting image data.
FIG. 7 is a conceptual diagram illustrating an embodiment of operation S111.
FIG. 8 is a conceptual diagram illustrating an embodiment of operation S111.
FIG. 9 is a conceptual diagram illustrating an embodiment of operation S112.
FIG. 10 is a conceptual diagram illustrating an exemplary gradient of a pixel line.
FIG. 11 is a conceptual diagram illustrating exemplary inflection pixels constituting image data.
FIG. 12 is a graph showing an exemplary relationship between a reference value used to determine inflection pixels and the number of inflection pixels.
FIG. 13 is a conceptual diagram illustrating an embodiment of operation S113.
FIG. 14 is a flowchart illustrating exemplary operations of an electronic device for processing image data.
FIG. 15 is a conceptual diagram illustrating an embodiment of operation S214.
FIG. 16 is a conceptual diagram illustrating an embodiment of operation S214.
FIG. 17 is a conceptual diagram illustrating a network system according to an embodiment of the present disclosure.

### DETAILED DESCRIPTION

Hereinafter, embodiments of the present disclosure will be described clearly and in detail to the extent that those skilled in the art to which the present disclosure pertains may easily realize the embodiments according to the present disclosure.

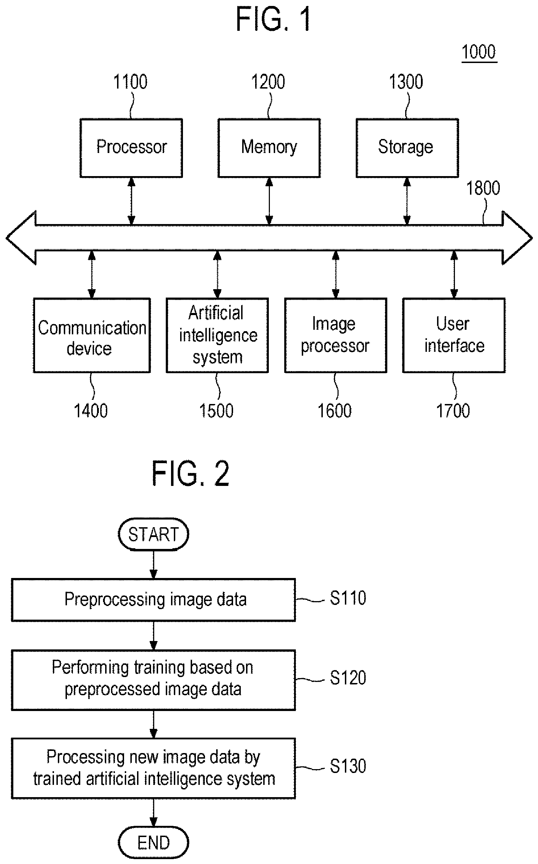

FIG. 1 is a block diagram illustrating an electronic device configured to process data according to an embodiment of the present disclosure.

Referring to FIG. 1, an electronic device 1000 may include a processor 1100, a memory 1200, a storage 1300, a communication device 1400, an artificial intelligence system 1500, an image processor 1600, a user interface 1700, and a bus 1800. For example, the electronic device 1000 may be implemented by at least one of the various types of electronic devices such as a portable communication device, a portable multimedia device, a wearable device, a personal computer device, a workstation, and the like, or a combination thereof.

However, the elements of the electronic device 1000 are not limited to the embodiment shown in FIG. 1. The electronic device 1000 may exclude one or more of the elements shown in FIG. 1 or further include at least one element not shown in FIG. 1. For example, the electronic device 1000 may further include various types of sensors for detecting a variety of physical energy from the outside of the electronic device 1000, a security module that operates to protect stored information from an external attacker, and the like.

The processor 1100 may control the overall operation of the electronic device 1000. For example, the processor 1100 may be implemented as a general-purpose processor, a dedicated processor, an application processor, or the like. The processor 1100 may process various operations for operating the electronic device 1000.

The processor 1100 may receive image data through the communication device 1400 and/or the user interface 1700. The processor 1100 may receive image data obtained by the image processor 1600.

The image data may be related to an image corresponding to an object or background outside the electronic device 1000. For example, the image data may indicate an image of a part or all of a living body such as a human body. For example, the image data may be obtained on the basis of radiation (e.g., an X-ray) irradiated on a part or all of the living body such as a human body. Hereinafter, although the image data representing an X-ray image of a part or all of the human body will be described by way of example to facilitate understanding, the embodiments of the disclosure are not limited thereto, and it will be understood that the image data can be obtained according to various methods on the basis of the image of any object or background.

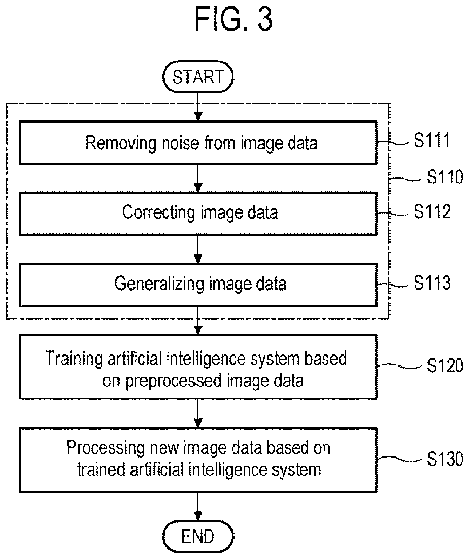

The processor 1100 may process the image data received from the image processor 1600 in order to produce image data to be used for the operation of the artificial intelligence system 1500. For example, the processor 1100 may perform a preprocessing operation to produce the image data to be used to train the artificial intelligence system 1500. Referring to FIGS. 2 and 3, exemplary methods of preprocessing the image data by the processor 1100 will be described in detail.

The memory 1200 may store data required for the operation of the electronic device 1000. For example, the memory 1200 may store image data processed or to be processed by the processor 1100 and/or the artificial intelligence system 1500. For example, the memory 1200 may include at least one of volatile memory such as static random access memory (SRAM), dynamic RAM (DRAM), synchronous DRAM (SDRAM), and the like, and/or non-volatile memory such as flash memory, phase-change RAM (PRAM), magneto-resistive RAM (MRAM), resistive RAM (ReRAM), ferro-electric RAM (FRAM), and the like.

The storage 1300 may store data regardless of a power supply. For example, the storage 1300 may store image data processed or to be processed by the processor 1100 and/or the artificial intelligence system 1500. For example, the storage 1300 may include at least one of various non-volatile memories such as flash memory, PRAM, MRAM, ReRAM, FRAM, and the like. Alternatively, the storage 1300 may include a removable memory such as a hard disk drive (HDD), a solid state drive (SSD), a secure digital (SD) card, and the like, and/or an embedded memory such as an embedded multimedia card (eMMC) and the like.

The communication device 1400 may be configured to communicate with other electronic devices and/or systems outside the electronic device 1000. The communication device 1400 may perform communication to obtain data to be used for operation of the processor 1100. For example, the communication device 1400 may receive image data to be used in a preprocessing operation of the processor 1100 from a server outside the electronic device 1000. The communication device 1400 may include a reception circuit configured to receive image data.

For example, the communication device 1400 may communicate with an external electronic device and/or system according to a wireless communication protocol such as long-term evolution (LTE), LTE Advance (LTE-A), code division multiple access (CDMA), wideband CDMA (WCDMA), Wireless Broadband (WiBro), wireless fidelity (Wi-Fi), Bluetooth, near-field communication (NFC), a global positioning system (GPS), and a global navigation satellite system (GNSS), and a wired communication protocol such as a universal serial bus (USB), a high definition multimedia interface (HDMI), recommended standard 232 (RS-232), and a plain old telephone service (POTS).

The artificial intelligence system 1500 may be trained on the basis of the data provided from the processor 1100. For example, the artificial intelligence system 150 may be trained according to various types of algorithms on the basis of the image data provided from the processor 1100. Thereafter, the electronic device 1000 may process newly input image data by the trained artificial intelligence system 1500.

For example, the artificial intelligence system 1500 may include an artificial neural network for implementing various types of machine learning. For example, the artificial intelligence system 1500 may include various types of hardware to implement the artificial neural network such as a convolutional neural network (CNN), a recurrent neural network (RNN), a generative adversarial network (GAN), and the like.

Alternatively, the artificial intelligence system 1500 may be configured to store program code for implementing the artificial neural network and to execute the program code. For example, the artificial intelligence system 1500 may include a separate processor (e.g., a neural processing unit (NPU), etc.) configured to execute machine learning. Alternatively, the artificial intelligence system 1500 may include a separate memory device (e.g., a memory device including memristor elements) configured to store data (e.g., weights, etc.) related to machine learning.

The artificial intelligence system 1500 may classify newly input image data and obtain new data from the classified image data. For example, the artificial intelligence system 1500 may be trained by the image data that is produced on the basis of an X-ray image of a part or all of a human body. The electronic device 1000 may classify newly input image data according to appropriate criteria (e.g., correlation between an X-ray image and the age of a human body, etc.) on the basis of the trained artificial intelligence system 1500.

The image processor 1600 may detect electromagnetic waves and radiation transmitted from the outside of the electronic device 1000, thereby producing image data. For example, the image processor 1600 may include an image sensor and an image signal processor for producing image data. For example, the image processor 1600 may receive an X-ray irradiated on a part or all of the human body, and obtain image data representing the image of the part or all of the human body on the basis of the received X-ray. The image processor 1600 may transmit the obtained image data to the processor 1100.

The user interface 1700 may relay communication between a user and the electronic device 1000. The user may input a command to the electronic device 1000 through the user interface 1700. For example, the electronic device 1000 may provide the user with information produced by the processor 1100 and the artificial intelligence system 1500 through the user interface 1700. For example, the electronic device 1000 may receive data to be used in the preprocessing operation of the processor 1100 through the user interface 1700. The user interface 1700 may include a reception circuit for receiving the image data to be used in the preprocessing operation of the processor 1100.

The bus 1800 may provide a path for communication between the elements of the electronic device 1000. For example, the elements of the electronic device 1000 may exchange data through the bus 1800 on the basis of various communication protocols.

Hereinafter, exemplary operations of preprocessing the image data by the processor 1100 will be described with reference to FIGS. 2 to 16.

FIG. 2 is a flowchart illustrating exemplary operations of an electronic device 1000 for processing image data.

Hereinafter, although it will be described that the operations described with reference to FIG. 2 are implemented as program code that can be executed by hardware and executed by a processing device such as the processor 1100, the embodiments of the disclosure are not limited thereto. For example, the operations to be described with reference to FIG. 2 may be implemented by various types of electronic circuits (e.g., various types of logic gates, application-specific integrated circuits (ASICs), field programmable gate arrays (FPGAs), etc.). Alternatively, the operations described with reference to FIG. 2 may be implemented by a combination of hardware such as firmware and software.

In operation S110, the processor 1100 of the electronic device 1000 may perform a preprocessing operation. For example, the processor 1100 may receive image data through various types of reception circuits (e.g., the reception circuit included in the communication device 1400 and/or the user interface 1700). The processor 1100 may preprocess the received image data. Hereinafter, an exemplary preprocessing operation will be described in detail with reference to FIG. 3.

In operation S120, the artificial intelligence system 1500 may receive the image data preprocessed in operation S110. The artificial intelligence system 1500 may be trained on the basis of the preprocessed image data. For example, the artificial intelligence system 1500 may calculate and update weights on the basis of image data repeatedly received from the processor 1100. The artificial intelligence system 1500 may store the calculated and updated weights. For example, the calculated and updated weights may be related to the operation of classifying image data (e.g., image data of an X-ray image representing a human hand) input to the artificial intelligence system 1500. The artificial intelligence system 1500 may perform calculations according to an activation function on the basis of the stored weights and the received image data.

In operation S130, the electronic device 1000 may receive new image data from the outside of the electronic device 1000. For example, the electronic device 1000 may receive image data input by the user through the user interface 1700. Alternatively, the electronic device 1000 may receive new image data from the outside of the electronic device 1000 through the communication device 1400.

The electronic device 1000 may process the new image data by the artificial intelligence system 1500 trained in operation S120. For example, the electronic device 1000 may classify newly input image data according to various criteria by the artificial intelligence system 1500. The electronic device 1000 may obtain new data on the basis of the classified image data. Thereafter, the electronic device 1000 may provide information related to the obtained data to the user through the user interface 1700.

For example, the electronic device 1000 may classify newly input image data by the artificial intelligence system 1500 trained on the basis of image data related to a human hand. The electronic device 1000 may obtain new information (e.g., information on the bone age corresponding to the X-ray image) on the basis of the classified image data.

FIG. 3 is a flowchart illustrating exemplary operations of the electronic device 1000 for processing image data. Referring to FIG. 3, operation S110 may include operations S111 to S113.

In operation S111, the processor 1100 may remove noise from the image data. For example, the image data may be related to a target object (e.g., a human hand) outside the electronic device 1000. The image data may include image data on a specific image that is not related to the target object. For example, noise may be produced due to a factor such as a dark current in the process of outputting the image data by the image processor 1600. Alternatively, the image data may include noise that is intentionally produced by the provider. The user of the electronic device 1000 may recognize the image displayed by the image data that is not related to the target object, among all the image data, as noise and remove the recognized noise from the image data.

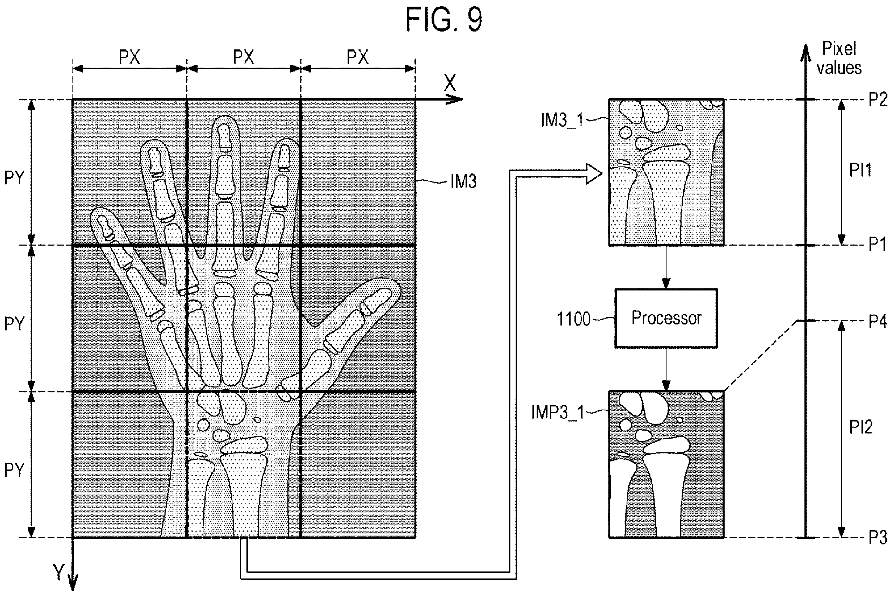

In operation S112, the processor 1100 may correct image data. For example, the processor 1100 may divide the image data into sub-image data respectively corresponding to a plurality of regions. The processor 1100 may correct pixel values of each piece of sub-image data included in the image data for visibility of the image corresponding to the image data. Operation S112 will be described in more detail with reference to FIG. 9.

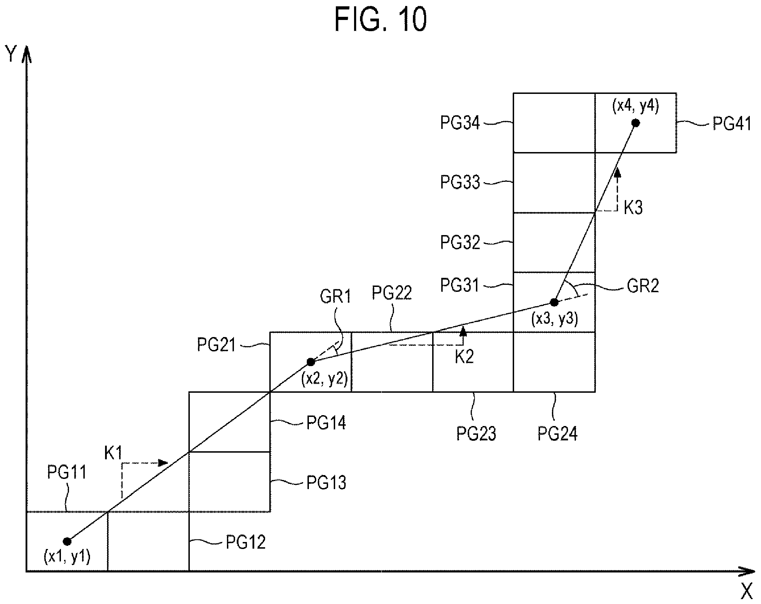

In operation S113, the processor 1100 may generalize the image data. For example, the image data may be configured in units of pixels, and the pixels constituting the image data may respectively correspond to specific coordinate values. The coordinate values of a pixel may indicate the position of the pixel in the entire image. The processor 1100 may select pixels that satisfy a specific condition from among the pixels of the image data. The processor 1100 may determine the type of image data on the basis of the selected pixels and change the coordinate values of the pixels of the corresponding image data such that the corresponding image data has a predetermined type. According to this, the image data may be generalized into one type of image data. For example, if image data on an X-ray image of a part or all of a human body (e.g., the hand) is received, the image data may be classified into one of two types (e.g., a right-handed type and a left-handed type). The processor 1100 may change the coordinate values of the image data classified into one of two types such that all the image data is classified into one type. Accordingly, the coordinate values of the image data representing an image of the right hand may be changed such that all the received image data indicate the image of the left-handed type in operation S113. Operation S113 will be described in more detail with reference to FIGS. 10 to 13.

The artificial intelligence system 1500 may be efficiently trained according to operation S120 being performed on the basis of the image data that is preprocessed in operations S111 to S113. In addition, the artificial intelligence system 1500 trained based on the preprocessed image data may provide improved performance in performing operation S130. Accordingly, the user may obtain information more suitable for the intended purpose on the basis of the trained artificial intelligence system 1500.

Although all operations S111 to S113 are described as being sequentially performed above, it will be understood that operations S111 to S113 may be performed in any order and that at least one of operations S111 to S113 may not be performed. For example, the processor 1100 may perform one or more of operations S111 to S113 in any order under the control of the user.

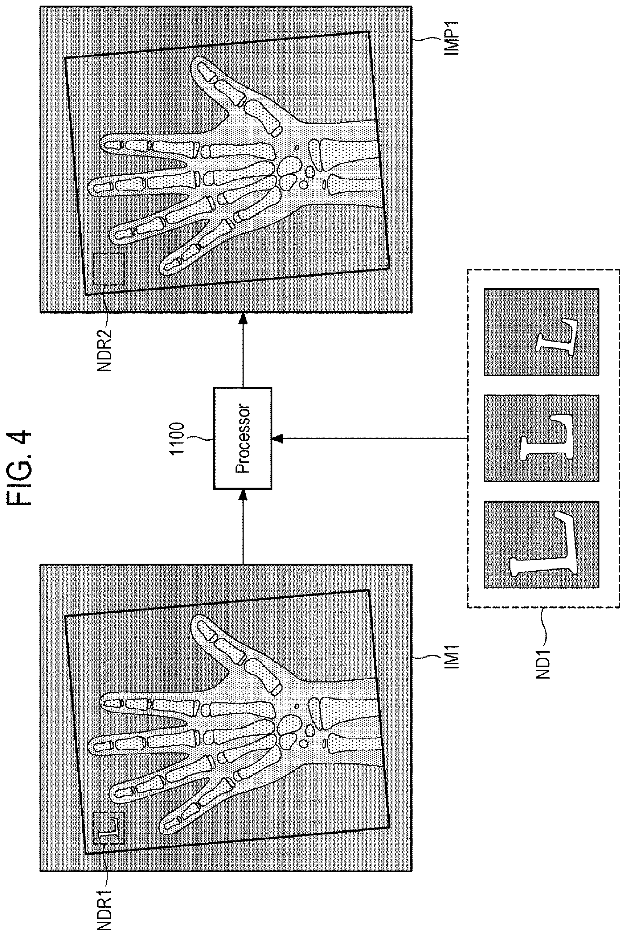

FIG. 4 is a conceptual diagram illustrating an embodiment of operation S111. The processor 1100 may perform operation S111 on image data IM1.

In the example shown in FIG. 4, the processor 1100 may receive image data IM1 and noise data ND1. For example, the electronic device 1000 may be provided with noise data ND1 through the user interface 1700 and/or the communication device 1400. The noise data ND1 may be related to the noise included in the image data IM1.

For example, image data including the image data IM1 may be repeatedly provided from various sources, and the provider of the image data may intentionally include noise in the image data in order to identify the image displayed by the image data. In the example shown in FIG. 4, the image "L" may be intentionally included in the image data by the provider of the image data.

In order to remove the noise that is intentionally included by the provider of the image data, the user of the electronic device 1000 may provide the electronic device 1000 with noise data (e.g., noise data ND1) that is the image data corresponding to the noise. Alternatively, the electronic device 1000 may be provided with noise data ND1 from another electronic device and system outside the electronic device 1000 by the communication device 1400. The electronic device 1000 may store the noise data ND1 in the memory 1200 and/or the storage 1300. In the example shown in FIG. 4, image data representing various images representing the shape of "L" may be provided as noise data ND1.

The processor 1100 may identify the noise included in the image data IM1 on the basis of the provided noise data ND1. The processor 1100 may determine, as noise, the image data included in a region NDR1 among the image regions corresponding to the image data IM1 on the basis of the noise data ND1. That is, the image data included in the region NDR1 may match the noise data ND1.

The processor 1100 may process the image data corresponding to the image of the region NDR1 in order to remove the noise. For example, the processor 1100 may adjust pixel values of the region NDR1. A pixel value of the image data may correspond to a specific value of the image represented by the image data. Hereinafter, although the pixel value of the image data will be described as indicating the contrast value of the image represented by the image data, the embodiments of the present disclosure are not limited thereto, and it will be understood that the specific value of the image displayed by the pixel value of the image data may vary widely. The pixel value of the image data may be a value within a specific range. For example, the pixel value may be one of the values from 0 to 255. For example, a magnitude of the range of pixel values may correspond to the quality of the image data. The designer of the electronic device 1000 may preconfigure the range of pixel values in consideration of the quality of the image data. For example, if the minimum pixel value corresponds to the darkest contrast value and if the maximum pixel value corresponds to the brightest contrast value, the processor 1100 may adjust the pixel values of the region NDR1 to the minimum value.

The processor 1100 may output the image data IMP1 having the adjusted pixel values. Thereafter, operations S112 and S113 may be performed on the basis of the output image data IMP1. The image region NDR2 represented by the image data IMP1 may correspond to the image region NDR1 represented by the image data IM1. That is, the image data of the region NDR2 may have the adjusted pixel values.

The image data displayed in the region NDR1 may not be related to the image of a target object (e.g., a human hand). Accordingly, in the case where the image data of a specific image displayed on the region ND1 is used for training the artificial intelligence system 1500, it may take much time to train the artificial intelligence system 1500 so as to output a meaningful result, or it may fail to train the same so as to output such a result. Accordingly, if the artificial intelligence system 1500 is trained on the basis of the image data IMP1 instead of the image data IM1, the performance of the artificial intelligence system 1500 may be improved.

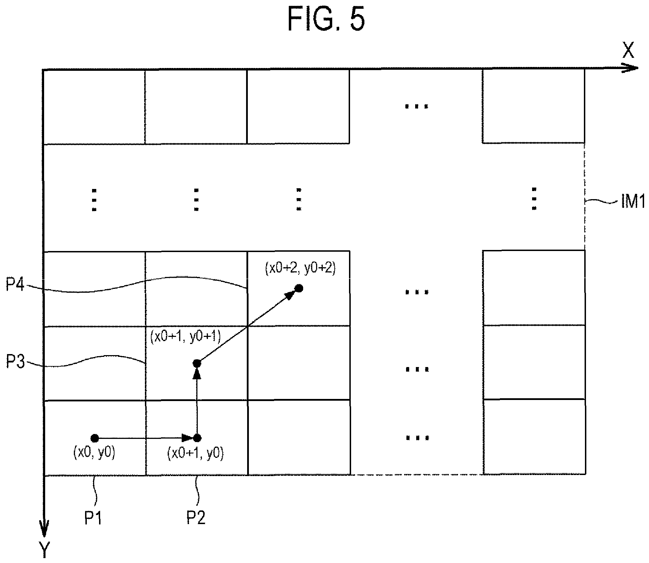

FIG. 5 is a conceptual diagram illustrating exemplary image data configured in units of pixels.

Referring to FIG. 5, the image data IM1 may include pixels having coordinate values on the X-axis and coordinate values on the Y-axis. However, a pixel having the coordinate values on the axes may mean that the image corresponding to the pixel is displayed at the position corresponding to the coordinate values on the image displayed on the basis of the image data, instead of meaning that the pixel is arranged in the physical space. Each of the pixels constituting the image data IM1 may have coordinate values on the X-axis and the Y-axis. For example, the coordinate value of the pixel P1 on the X-axis may be x0, and the coordinate value of the pixel P1 on the Y-axis may be y0.

The pixels constituting the image data IM1 may be adjacent to each other. Hereinafter, the configuration in which the pixels are adjacent to each other in the present specification means that the difference between the coordinate values of the pixels on the X-axis is a unit value (e.g., "1"), that the difference between the coordinate values of the pixels on the Y-axis is a unit value, or that both the difference between the coordinate values of the pixels on the X-axis and the difference between the coordinate values thereof on the Y-axis are unit values. For example, the coordinate value of a pixel P2 on the X-axis may be x0+1, and the coordinate value thereof on the Y-axis may be y0. Since the difference between the coordinate value of the pixel P1 on the X-axis and the coordinate value of the pixel P2 on the X-axis is a unit value of 1, the pixel P1 and the pixel P2 may be expressed as being adjacent to each other.

For example, the coordinate value of a pixel P3 on the X-axis may be x0+1, and the coordinate value thereof on the Y-axis may be y0+1. Since the difference between the coordinate value of the pixel P2 on the Y-axis and the coordinate value of the pixel P3 on the Y-axis is a unit value of 1, the pixel P2 and the pixel P3 may be expressed as being adjacent to each other. For example, the coordinate value of a pixel P4 on the X-axis may be x0+2, and the coordinate value thereof on the Y-axis may be y0+2. Since the difference between the coordinate value of the pixel P4 on the X-axis and the coordinate value of the pixel P3 on the X-axis is a unit value of 1, and since the different between the coordinate value of the pixel P4 on the Y-axis and the coordinate value of the pixel P3 on the Y-axis is a unit value of 1, the pixel P3 and the pixel P4 may be expressed as being adjacent to each other.

Although it has been described in FIG. 5 that the unit value is 1, it will be understood that the unit value may be variously configured by the designer of the electronic device 1000. Since the pixel P1 and the pixel P2 are adjacent to each other, the pixel P2 and the pixel P3 are adjacent to each other, and the pixel P3 and the pixel P4 are adjacent to each other, the pixels P1 to P4 may be expressed as a group of adjacent pixels.

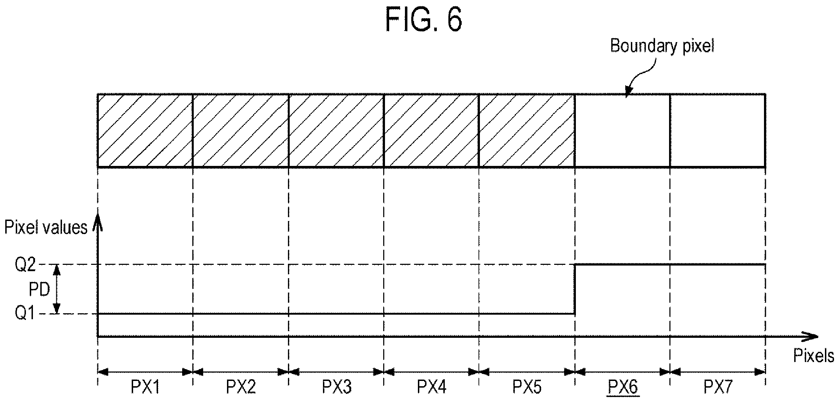

FIG. 6 is a conceptual diagram illustrating exemplary boundary pixels constituting image data. In the present disclosure, one pixel line may be defined by sequentially adjacent boundary pixels. The image represented by the pixel line may correspond to a boundary line between the image of a target object (e.g., a human hand) and a background image among the entire image of image data.

In the example shown in FIG. 6, pixels PX1 to PX7 may be a group of adjacent pixels among the pixels displaying the image data. The respective pixels PX1 to PX7 may have pixel values. For example, the respective pixels PX1 to PX5 may have a pixel value Q1, and the respective pixels PX6 and PX7 may have a pixel value Q2. Q1 may be less than Q2, and the difference between Q1 and Q2 may be PD.

Although it will be described that the image displayed by the pixels PX1 to PX5 having the smaller pixel value Q1 is relatively dark in contrast (the patterned pixels in FIG. 6) and that the image displayed by the pixels PX6 and PX7 having the larger pixel value Q2 is relatively bright in contrast (the pixels that are not patterned in FIG. 6), it will be understood that the relationship between the pixel values and the contrast values may be variously changed.

The boundary pixel may be determined on the basis of a difference between the pixel values of adjacent pixels. For example, the processor 1100 may calculate a difference between the pixel values of adjacent pixels. The processor 1100 may compare the difference with a threshold value. If the difference is equal to or greater than the threshold value, the processor 1100 may determine one of the pixels adjacent to each other as a boundary pixel.

For example, the threshold value may be determined in consideration of the distribution of the pixel values of the image data. The threshold value may be related to the number of boundary pixels determined in the image data. The designer of the electronic device 1000 may appropriately configure the threshold value such that the intended number of boundary pixels is included in the pixels representing the image data.

In the example shown in FIG. 6, the difference PD between the pixel value Q1 of the pixel PX5 and the pixel value Q2 of the pixel PX6, which is adjacent to the pixel PX5, may be greater than or equal to the threshold value configured by the designer. Accordingly, the processor 1100 may determine one of the adjacent pixels PX5 and PX6 as a boundary pixel. For example, the processor 1100 may determine the pixel PX6 having a larger pixel value Q2 as a boundary pixel.

However, it will be understood that the method for determining a boundary pixel on the basis of a difference between the pixel values of adjacent pixels may be variously changed and modified. For example, the processor 1100 may determine the pixel PX5 having a smaller pixel value, among the adjacent pixels PX5 and PX6, as a boundary pixel.

Alternatively, the processor 1100 may further determine at least one pixel sequentially adjacent to at least one of the pixels PX5 and PX6 as a boundary pixel. For example, if the difference PD between the pixel values of the pixels PX5 and PX6 is equal to or greater than the threshold value, the processor 1100 may determine, as boundary pixels, the pixel with having a larger pixel value, among the adjacent pixels PX5 and PX6, and the pixel PX7 adjacent to the pixel PX6.

Alternatively, if the difference PD between the pixel values of the pixels PX5 and PX6 is equal to or greater than the threshold value, the processor 1100 determine, as boundary pixels, the pixel PX5 having a smaller pixel value, among the pixels PX5 and PX6 adjacent to each other, and at least one pixel sequentially adjacent to the pixel PX5. That is, the pixels PX1 to PX5 may be determined as boundary pixels, the pixels PX2 to PX5 may be determined as boundary pixels, the pixels PX3 to PX5 may be determined as boundary pixels, or the pixels PX4 and PX5 may be determined as boundary pixels.

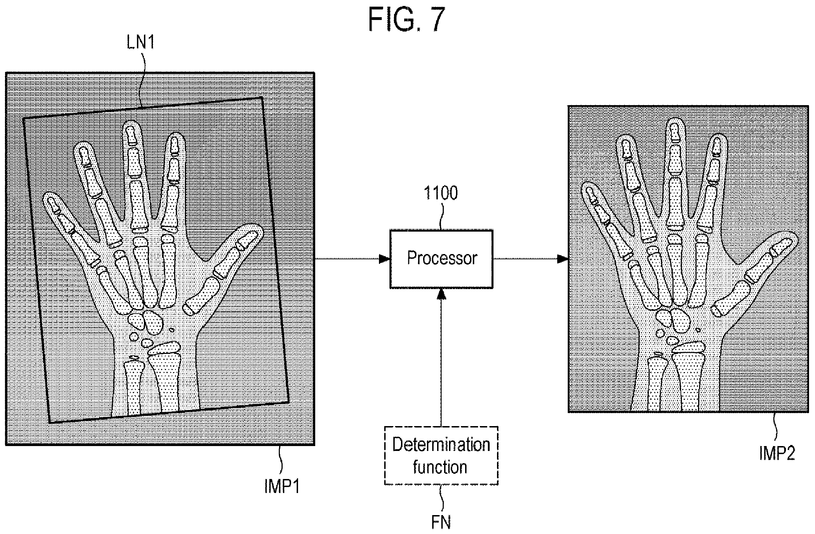

FIG. 7 is a conceptual diagram illustrating an embodiment of operation S111. As described above, the processor 1100 may perform operation S111 on image data IMP1.

As described with reference to FIG. 6, the processor 1100 may determine boundary pixels among the pixels constituting the image data IMP1. As described with reference to FIG. 5, boundary pixels that are included in the image data IMP1 may be adjacent to each other. The pixels constituting the image data IMP1 may include a group of sequentially adjacent boundary pixels (hereinafter referred to as a "pixel line"). For example, the pixels of the image data IMP1 may include a pixel line LN1. The differences between the pixel values of the pixel line LN1 and the pixel values of other pixels adjacent to the pixel line LN1 may be greater than or equal to a threshold value.

The processor 1100 may call a function for determining an array of the pixel line (hereinafter referred to as a "determination function" FN). For example, the processor 1100 may call a function stored in the memory 1200, the storage 1300, and/or a buffer (not shown). The image displayed by the pixel line LN1 may have a specific form according to the array of the pixel line LN1.

Hereinafter, the array of the pixel line LN1 may indicate a pattern of the image data determined by the coordinate values of the boundary pixels rather than a physical array of the boundary pixels constituting the pixel line LN1. For example, the array of the boundary pixels may correspond to a specific form/pattern/shape of the image to be provided by the display device or the like on the basis of the image data indicated by the boundary pixels.

Alternatively, the array, which is a value or a group of values indicating the relationship between the boundary pixels, may be calculated on the basis of the coordinate values of the boundary pixels. For example, the array may be related to the gradients calculated on the basis of the differences between the coordinate values of boundary pixels and/or the differences between the gradients. Definition of the gradients and the difference between the gradients will be described in more detail later with reference to FIG. 10, so a description thereof will be omitted here.

The processor 1100 may determine the array of the pixel line LN1 on the basis of the determination function FN, and extract image data (hereinafter referred to as "region image data") of the region divided by the pixel line LN1 if the determined array corresponds to a reference array. The processor 1100 may output the extracted region image data IMP2. Thereafter, operations S112 and S113 may be performed on the basis of the region image data IMP2.

In the example shown in FIG. 7, the pixel line LN1 may have an array corresponding to a rectangular image. If the rectangular image indicated by the pixel line LN1 is not a target image required for the user, the image may be perceived as noise by the user. Accordingly, the processor 1100 may process the image data IMP1 based on the pixel line LN1 that is recognized as noise by the user.

For example, the processor 1100 may call a determination function FN for determining the array corresponding to the rectangular image. The processor 1100 may perform calculations according to the determination function FN on the basis of the coordinates of the pixel line LN1. The processor 1100 may determine whether or not the array of the pixel line LN1 corresponds to the rectangular image on the basis of the calculation performed.

If it is determined that the array of the pixel line LN1 corresponds to the rectangular image, the processor 1100 may extract region image data IMP2 indicated by the pixels in the region divided by the pixel line LN1. The processor 1100 may output the extracted region image data IMP2.

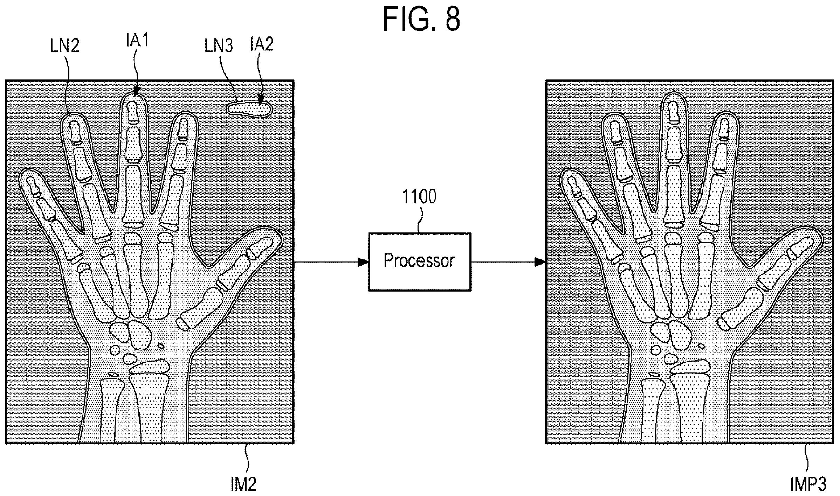

FIG. 8 is a conceptual diagram illustrating an embodiment of operation S111. The processor 1100 may perform operation S111 on image data IM2.

The image data IM2 may include pixel lines LN2 and LN3. The image data IM2 may include image data on the regions IA1 and IA2 divided by the pixel lines LN2 and LN3. The processor 1100 may determine whether or not the image data on the region IA1 divided by the pixel line LN2 and the image data on the region IA2 divided by the pixel line LN3 include noise.

In the present disclosure, the regions IA1 and IA2 indicate a group of pixels specified on the basis of the coordinate values of the pixels, instead of physical regions. For example, the image displayed in the region IA1 may be separated from the image (e.g., a background image) displayed in the region other than the region IA1 by the image displayed by the pixel line LN2 in the entire image.

In an embodiment, the processor 1100 may determine noise from the image data on the basis of a length of the pixel line. Specifically, the processor 1100 may calculate a length of the pixel line LN2 and a length of the pixel line LN3. The length of a specific pixel line may be related to the number of pixels constituting the pixel line rather than a physical length. The longer the length of the pixel line (i.e., the larger the number of pixels included in the pixel line), the longer the length of the image displayed by the image data of the pixel line may be.

For example, the processor 1100 may count the number of boundary pixels included in each of the pixel lines LN2 and LN3 in order to calculate the lengths of the pixel lines LN2 and LN3. The processor 1100 may calculate the length of each of the pixel lines LN2 and LN3 on the basis of the counted number of boundary pixels.

The processor 1100 may determine whether or not the image data corresponding to the pixels of the regions IA1 and IA2 is noise on the basis of the calculated lengths of the pixel lines LN2 and LN3. For example, the processor 1100 may determine that the image data included in the regions divided by the pixel lines other than the pixel line having the longest length is noise.

In the example shown in FIG. 8, the pixel line LN2 may be longer than the pixel line LN3. The processor 1100 may determine that the image data of the region IA1 divided by the pixel line LN2 having a longer length is the image data corresponding to the image of a target object. That is, the processor 1100 may determine that the image data of the region IA1 divided by the pixel line LN2 having a longer length is not noise. In addition, the processor 1100 may determine, as noise, the image of the region IA2 divided by the pixel line LN3 having a shorter length.

In an embodiment, the processor 1100 may determine noise from the image data on the basis of the areas of the regions divided by the pixel lines. Specifically, the processor 1100 may calculate the areas of the regions IA1 and IA2 (hereinafter referred to as "areas of regions IA1 or IA2") divided by the pixel lines LN2 and LN3. In the present specification, the area of a region may be related to the number of pixels included in the region, instead of indicating the area of a physical region. For example, the processor 1100 may count the number of pixels included in each of the regions IA1 and IA2. The processor 1100 may calculate areas of the images corresponding to the image data of the regions IA1 and IA2 on the basis of the counted number of pixels.

The processor 1100 may determine whether or not the image data displayed by the pixels included in the regions IA1 and IA2 is noise on the basis of the calculated areas of the regions IA1 and IA2. For example, the processor 1100 may determine that the image data of the regions other than the region having the largest area among the regions divided by the pixel lines is noise. In the example shown in FIG. 8, the area of the region IA1 may be greater than the area of the region IA2. The processor 1100 may determine that the image data of the region IA2 having a smaller region is noise.

The processor 1100 may adjust the pixel values of the pixels representing the image of the region IA2 to remove noise. For example, in the case where the minimum pixel value corresponds to the darkest contrast value and where the maximum pixel value corresponds to the brightest contrast value, the processor 1100 may adjust the pixel values of the region IA2 determined as noise to the minimum value. The processor 1100 may output image data IMP3 including the adjusted pixel values. Thereafter, operations S112 and S113 may be performed on the basis of the image data IMP3.

FIG. 9 is a conceptual diagram illustrating an embodiment of operation S112. The processor 1100 may perform operation S112 on image data IM3.

The processor 1100 may divide the region of the image data IM3 into a plurality of regions. Each of the plurality of divided regions may indicate sub-image data. For example, the processor 1100 may divide the image data IM3 on the basis of coordinate values of pixels representing the image data IM3. Sub-image data divided from the image data IM3 may not overlap each other. Accordingly, regions of the images displayed by the image data may not overlap each other. The sum of the sub-image data divided from the image data IM3 may be substantially the same as the image data IM3.

In the example shown in FIG. 9, the processor 1100 may divide the image data IM3 such that the pixels of the image data IM3 are divided into three regions on the X-axis and such that the pixels of the image data IM3 are divided into three regions on the Y-axis. Accordingly, the image data IM3 may be divided into sub-image data corresponding to nine regions. The sub-image data may have a size of "PX pixels X PY pixels." Although the embodiment for dividing the region of the image data IM3 into sub-image data having the same size has been described, it will be understood that a method for dividing the image data IM3 may be variously changed and/or modified.

Thereafter, the processor 1100 may correct pixel values of the divided sub-image data. In the example shown in FIG. 9, the processor 1100 may correct pixel values of sub-image data IM3_1 included in the image data IM3. For example, the processor 1100 may scale the pixel values of the sub-image data IM3_1. For example, the processor 1100 may multiply the pixel values of the sub-image data IM3_1 by a scaling factor (e.g., a natural number of 2 or more).

Alternatively, the processor 1100 may subtract a fixed value from the pixel values of the sub-image data IM3_1 or add a fixed value to the pixel values. Alternatively, the processor 1100 may change the pixel values less than or equal to a specific value, among the pixel values of the sub-image data IM3_1, to a minimum value. Alternatively, the processor 1100 may change the pixel values less than or equal to a specific value, among the pixel values of the sub-image data IM3_1, to a maximum value. For example, in the case where the image data is expressed as 8-bit data, the minimum value of the pixel value may be 0 and the maximum value thereof may be 255.

In the example shown in FIG. 9, the pixel values of the sub-image data IM3_1 may have a range PI1 of P1 to P2. The processor 1100 may adjust the pixel values of the sub-image data IM3_1 such that the pixel values of the sub-image data IM3_1 have a range PI2 of P3 to P4 on the basis of various algorithms. A magnitude of the range PI2 may be greater than a magnitude of the range PI1.

In FIG. 9, since the range PI2 is greater than the range PI1, the contrast value of the image displayed by the sub-image data IMP3_1 may be greater than the contrast value of the image displayed by the sub-image data IM3_1.

In FIG. 9, although it is illustrated that the ranges PI1 and PI2 do not overlap each other and that the pixel values of the range PI1 are greater than the pixel values of the range PI2, it will be understood that the relationship between the ranges PI1 and PI2 may be variously changed/modified. For example, a portion of the range PI1 may overlap a portion of the range PI2. Alternatively, the range PI2 may include the range PI1. Alternatively, the ranges PI1 and PI2 may not overlap each other, and the pixel values of the range PI2 may be greater than the pixel values of the range PI1.

As the contrast value of the image increases, the artificial intelligence system 1500 may obtain accurate image data on the object included in the image (e.g., a skeletal shape included in the X-ray image or the like). Accordingly, the artificial intelligence system 1500 may clearly determine the image data representing the target, and may be trained on the basis of the determined the image data.

The artificial intelligence system 1500 may be trained on the basis of the image data IMP3_1 as well as the image data IM3_1. The image data IMP3_1 to be used to train the artificial intelligence system 1500 may be further produced by operation S112, and the artificial intelligence system 1500 may be trained on the basis of a larger amount of image data, thereby improving the performance of the artificial intelligence system 1500.

The processor 1100 may perform operation S112 on all sub-image data included in the image data IM3 according to a method similar to the method described with reference to FIG. 9. Accordingly, operation S112 may be performed on the entire image data IM3 by the processor 1100. Thereafter, operations S111 and S113 may be performed on the basis of the image data processed in operation S112.

FIG. 10 is a conceptual diagram illustrating an exemplary gradient of a pixel line.

Referring to FIG. 10, pixels PG11 to PG14, pixels PG21 to PG24, pixels PG31 to PG34, and pixel PG41 may be sequentially adjacent to each other. Accordingly, the pixels PG11 to PG14, the pixels PG21 to PG24, the pixels PG31 to PG34, and the pixel PG41 may constitute a pixel line.

A coordinate value of the pixel PG11 on the X-axis may be x1, and a coordinate value thereof on the Y-axis may be y1. A coordinate value of the pixel PG21 on the X-axis may be x2, and a coordinate value thereof on the Y-axis may be y2. A coordinate value of the pixel PG31 on the X-axis may be x3, and a coordinate value thereof on the Y-axis may be y3. A coordinate value of the pixel PG41 on the X-axis may be x4, and a coordinate value thereof on the Y-axis may be y4.

The processor 1100 may calculate gradients of the pixel line. For example, the processor 1100 may calculate a gradient of the pixel line on the basis of coordinate values of sequentially adjacent N pixels (where N is a natural number) among the pixels included in the pixel line. For example, if N is 5, the processor 1100 may calculate, as a gradient, a rate of change in the coordinate values between a first pixel and a fifth pixel among five sequentially adjacent pixels. The designer may preset N in consideration of various conditions (e.g., performance of the processor and the like) and it will be understood that N may be variously changed according to the designer's setting.

For example, the processor 1100 may calculate, as a gradient K1 of the pixel line, a rate of change between the first pixel PG11 and the fifth pixel PG21 among the pixels PG11 to PG21. That is, the processor 1100 may calculate, as a gradient K1 of the pixel line, (y2-y1)/(x2-x1) between the pixels PG11 and PG21.

For example, the processor 1100 may calculate, as a gradient K2 of the pixel line, a rate of change between the first pixel PG21 and the fifth pixel PG31 among the pixels PG21 to PG31. That is, the processor 1100 may calculate, as a gradient K2 of the pixel line, (y3-y2)/(x3-x2) between the pixels PG21 and PG31.

For example, the processor 1100 may calculate, as a gradient K3 of the pixel line, a rate of change between the first pixel PG31 and the fifth pixel PG41 among the pixels PG31 to PG41. That is, the processor 1100 may calculate, as a gradient K3 of the pixel line, (y4-y3)/(x4-x3) between the pixels PG31 and PG41.

The processor 1100 may calculate a difference between the gradients, that is, a change in the gradients. In the example shown in FIG. 10, the processor 1100 may calculate a difference (a value corresponding to an angle GR1) between the gradient K1 of the pixels PG11 and PG21 and the gradient K2 of the pixels PG21 and PG31. The processor 1100 may calculate a difference (a value corresponding to an angle GR2) between the gradient K2 of the pixels PG21 and PG31 and the gradient K3 of the pixels PG31 and PG41.

If a change in the gradient is equal to or greater than a reference value, the processor 1100 may determine a pixel having coordinate values in which the gradient changes as an inflection pixel. In the present disclosure, the inflection pixel may indicate a pixel corresponding to an inflection point of a pixel line when it is assumed as a continuous line.

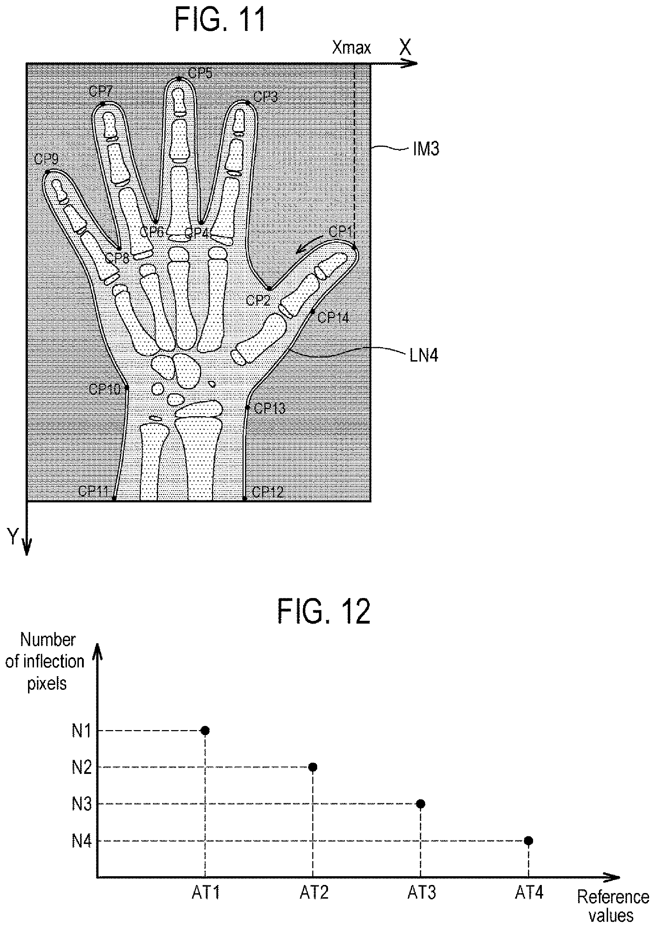

The processor 1100 may change a reference value in consideration of the number of inflection pixels included in the image data. Exemplary operations of changing a reference value in consideration of the number of inflection pixels included in the image data will be described with reference to FIG. 12.

FIG. 11 is a conceptual diagram illustrating exemplary inflection pixels constituting image data.

Referring to FIG. 11, pixels of image data IM3 may include a pixel line LN4. The processor 1100 may calculate gradients on the pixel line LN4. The processor 1100 may determine inflection pixels on the basis of the calculated gradients. In the example shown in FIG. 11, the pixel line LN4 may include 14 inflection pixels.

The processor 1100 may index the inflection pixels of the pixel line LN4. The processor 1100 may index the inflection pixels in a consecutive order. For example, the processor 1100 may index the inflection pixels on the basis of the coordinate values of the inflection pixels on the X-axis and the coordinate values thereof on the Y-axis.

In the example shown in FIG. 11, the processor 1100 may index the inflection pixel having the largest coordinate value "Xmax," among the coordinate values on the X-axis, as "CP1." The processor 1100 may search for inflection pixels along the pixel line PN4 from the inflection pixel "CP1." The processor 1100 may index sequentially searched inflection pixels as "CP2" to "CP14," respectively.

For example, the processor 1100 may determine inflection pixels of the pixel line LN4 along the direction in which the coordinate values on the X-axis decrease (i.e., the counterclockwise direction in FIG. 11) from the inflection pixel "CP1," and index the inflection pixels as "CP2" to "CP14" according to the determined order.

FIG. 12 is a graph showing an exemplary relationship between a reference value used to determine inflection pixels and the number of inflection pixels.

In the example shown in FIG. 12, if a change in the gradient of a specific boundary pixel on the pixel line LN4 is greater than or equal to a reference value "AT1," the processor 1100 may determine the boundary pixel to be an inflection pixel. In this case, N1 boundary pixels may be determined to be inflection pixels on the pixel line LN4. Similarly, "N2," "N3," and "N4" boundary pixels may be determined as inflection pixels to respectively correspond to reference values "AT2," "AT3," and "AT4." As the reference value used to determine the inflection pixel among the pixels of the image data IM3 increases, the number of inflection pixels may decrease.

The processor 1100 may determine the number of inflection pixels by continuously changing the reference value in the image data IM3 until the reference number of inflection pixels is determined. For example, the designer of the electronic device 1000 may set the reference number of inflection pixels to 14. The processor 1100 may determine the number of inflection pixels in the image data IM3 while gradually increasing the reference value such that 14 inflection pixels are determined in the image data. Accordingly, the processor 1100 may determine "14" inflection pixels in the image data IM3 to correspond to the reference value.

Thereafter, if new image data is repeatedly received, the processor 1100 may determine the number of inflection pixels while gradually increasing the reference value such that the reference number of inflection pixels is determined in the new image data. Accordingly, a preset reference number of inflection pixels may be determined even in any newly received image data. That is, the number of inflection pixels determined in the image data by the processor 1100 may be fixed.

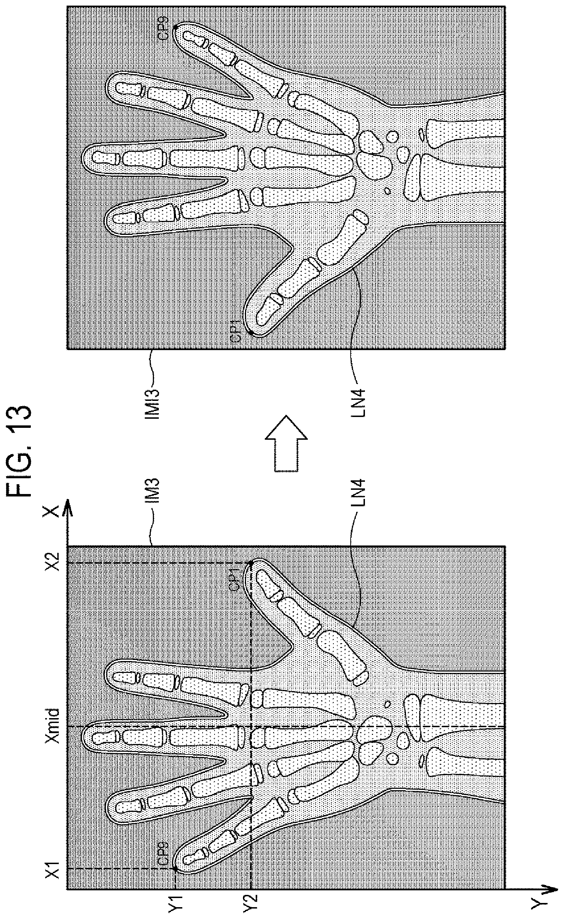

FIG. 13 is a conceptual diagram illustrating an embodiment of operation S113.

The processor 1100 may obtain coordinate values of inflection pixels of the image data IM3. The processor 1100 may determine reference pixels on the basis of the coordinate values of the inflection pixels. For example, the processor 1100 may determine, as reference pixels, an inflection pixel "CP1" having the largest coordinate value on the X-axis and an inflection pixel "CP9" having the smallest coordinate value on the X-axis.

Although an embodiment in which the inflection pixel "CP1" and the inflection pixel "CP9" are determined as reference pixels will be described with reference to FIG. 13, the method for determining the reference pixels may be variously changed and/or modified. For example, the processor 1100 may compare the coordinate values of the inflection pixels with each other on the basis of various algorithms, and determine specific inflection pixels representing the characteristics of the image data IM3 as reference pixels on the basis of the comparison result.

The processor 1100 may compare a coordinate value of the inflection pixel "CP1" on the Y-axis with a coordinate value of the inflection pixel "CP9" on the Y-axis. If the coordinate value of the inflection pixel "CP1" on the Y-axis is smaller than the coordinate value of the inflection pixel "CP9" on the Y-axis, the processor 1100 may change the overall coordinate values of the pixels constituting the image data IM3.

For example, the processor 1100 may invert the coordinate values of the pixels, which constitute the image data IM3, on the X-axis on the basis of an intermediate value Xmid of the coordinate values on the X-axis. The processor 1100 may output image data IMI3 represented by the pixels having inverted coordinate values. Thereafter, operations S111 and S112 may be performed on the basis of the image data IMI3.

The processor 1100 may process all newly received image data according to a method similar to the method described with reference to FIG. 13. Accordingly, all image data received by the processor 1100 may be generalized. For example, image data IM3 on an X-ray image related to a human hand may be received. The processor 1100 may classify the image data into a first type (e.g., a right-handed type) if the coordinate value of the inflection pixel "CP1" on the Y-axis is greater than the coordinate value of the inflection pixel "CP9" on the Y-axis, and classify the image data into a second type (e.g., a left-handed type) if the coordinate value of the inflection pixel "CP9" on the Y-axis is greater than the coordinate value of the inflection pixel "CP1" on the Y-axis.

If the first type of image data and the second type of image data are received, the processor 1100 may change coordinate values of the second type of image data according to an operation similar to operation S113. The image data having changed coordinate values may be reclassified into the first type. Accordingly, all the image data generalized by the processor 1100 may be classified into the first type. Similarly, the processor 1100 may generalize the received image data such that all the image data is classified into the second type.

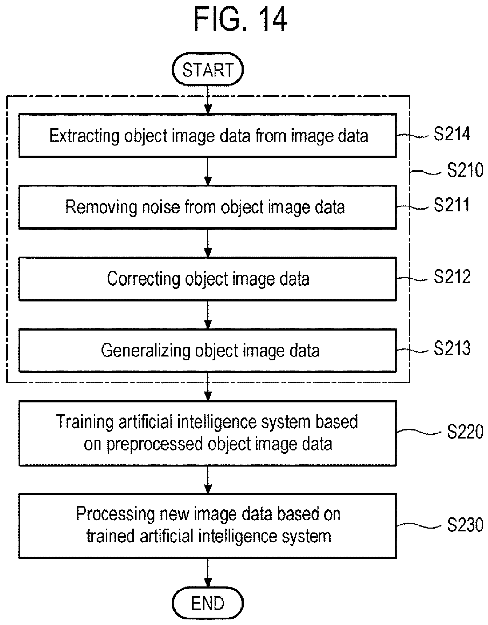

FIG. 14 is a flowchart illustrating exemplary operations of the electronic device 1000 for processing image data.

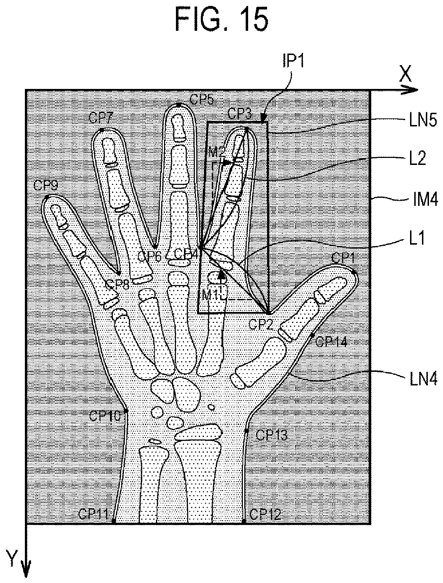

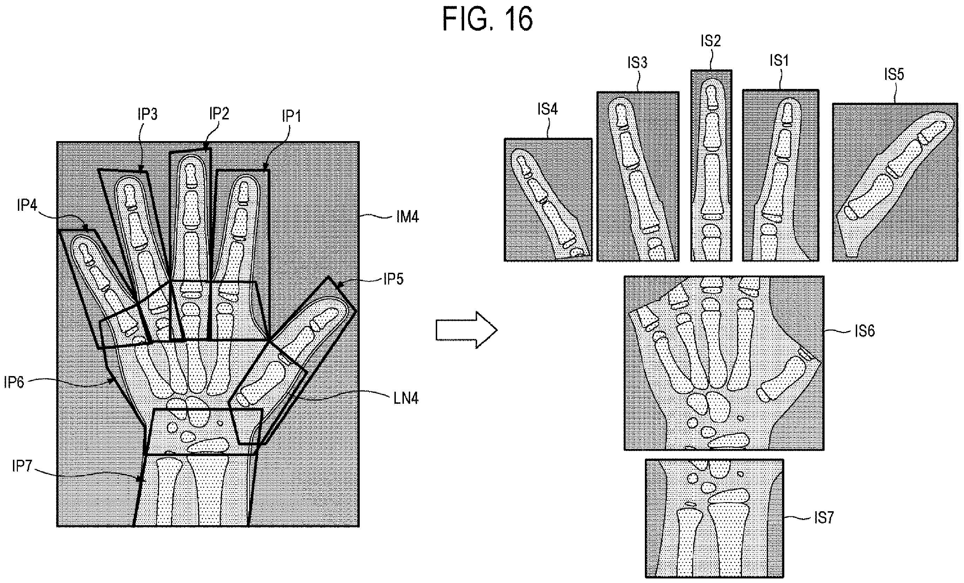

In operation S214, the processor 110 may extract object image data from the received image data. For example, the processor 1100 may divide image data into sub-image data, and select, as object image data, sub-image data satisfying an appropriate condition from among the divided sub-image data. The exemplary operation S214 will be described in more detail with reference to FIGS. 15 and 16.

Comparing FIG. 14 with FIG. 3, operations S110 to S130 respectively correspond to operations S210 to S230 and operations S111 to S113 respectively correspond to operations S211 to S213, so duplicate descriptions thereof will be omitted below. However, the processor 1100 may perform operations S211 to S213, S220, and S230 on the object image data extracted in operation S214.

Operation S214 may be performed before operations S211 to S213 are performed. Although all of operations S211 to S213 are illustrated as being performed in sequence to facilitate understanding, it will be understood that operations S211 to S213 may be performed in any sequence and that at least one of operations S211 to S213 may not be performed. For example, the processor 1100 may perform one or more of operations S211 to S213 in any order under the control of the user.

FIG. 15 is a conceptual diagram illustrating an embodiment of operation S214.

In the example shown in FIG. 15, the processor 1100 may determine 14 inflection pixels among the pixels of the image data IM4 and index the determined inflection pixels as "CP1" to "CP14." The processor 1100 may determine a pixel line LN5 on the basis of inflection pixels "CP2" to "CP4."

For example, the processor 1100 may calculate a distance L1 between the inflection pixel "CP2" and the inflection pixel "CP4" on the basis of the coordinate values of the inflection pixels "CP2" and "CP4." The processor 1100 may calculate a distance L2 between the inflection pixel "CP3" and the inflection pixel "CP4" on the basis of the coordinate values of the inflection pixels "CP3" and "CP4." In this specification, the distance between inflection pixels may indicate a value calculated on the basis of coordinate values of the inflection pixels rather than a physical distance.

For example, the processor 1100 may calculate a gradient M1 from the inflection pixel "CP2" to the inflection pixel "CP4" on the basis of the coordinate values of the inflection pixels "CP2" and "CP4." The processor 1100 may calculate a gradient M2 from the inflection pixel "CP3" to the inflection pixel "CP4" on the basis of the coordinate values of the inflection pixels "CP3" and "CP4." The processor 1100 may determine a pixel line LN5 on the basis of the distances L1 and L2, and the gradients M1 and M2. The processor 1100 may extract image data of a region IP1 divided by the pixel line LN5 as sub-image data of the image data IM4.

FIG. 16 is a conceptual diagram illustrating an embodiment of operation S214.

The processor 1100 may extract sub-image data from the image data IM4 on the basis of a method similar to the method described with reference to FIG. 15. The processor 1100 may determine pixel lines on the basis of the inflection pixels and extract image data of the pixels included in regions IP1 to IP7 separated by the pixel lines as sub-image data. The processor 1100 may output sub-image data IS1 to IS7 respectively corresponding to the regions IP1 to IP7 from the image data IM4.

The processor 1100 may select object image data from among the sub-image data IS1 to IS7. For example, the image data IM4 may represent an X-ray image of a human hand. The user may control the electronic device 1000 to select sub-image data for a part of the hand image that meets a specific purpose as object image data. The processor 1100 may select object image data from among the sub-image data IS 1 to IS7 under the control of the user.

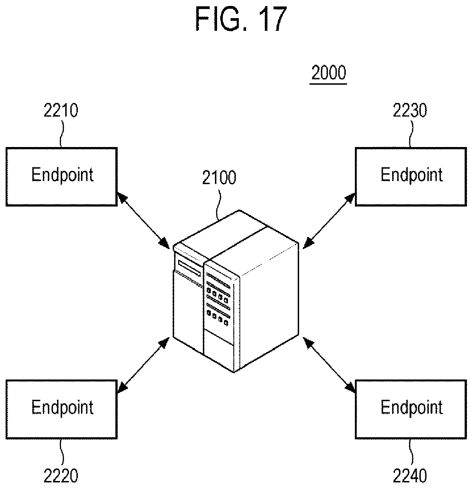

FIG. 17 is a conceptual diagram illustrating a network system according to an embodiment of the present disclosure.

Referring to FIG. 17, a network system 2000 may include a server 2100 and endpoints 2210 to 2240. Each of the endpoints 2210 to 2240 may include an electronic device similar to the electronic device 1000.

The endpoints 2210 to 2240 may exchange a variety of data with the server 2100. For example, the endpoints 2210 to 2240 may receive image data to be used for training the artificial intelligence system from the server 2100. Alternatively, the endpoints 2210 to 2240 may receive, from the server 2100, a variety of data (e.g., the noise data ND1, the image data IM1, the image data IMP1, the image data IMP2, the image data IM3, the image data IM4, and the like) used in operations S111 to S113, S120, S130, S211 to S214, S220, and S230.

Each of the endpoints 2210 to 2240 may process a variety of data by a trained artificial intelligence system. For example, each of the endpoints 2210 to 2240 may receive image data on an X-ray image representing a part or all of a human body, and obtain information related to the human body on the basis of the received image data. The endpoints 2210 to 2240 may exchange information via the server 2100.

Although the embodiment of the network system 2000 configured in a star type has been described with reference to FIG. 17, it will be understood that the type of the network system 2000 may be variously changed and/or modified. For example, the network system 2000 may be configured in at least one of a bus sharing type, a ring type, a mesh type, and the like.

According to an embodiment of the present disclosure, image data can be preprocessed to train the artificial intelligence system, and the artificial intelligence system can be efficiently trained on the basis of the preprocessed image data.

The above descriptions are specific embodiments for carrying out the present disclosure. The present disclosure encompasses the embodiments that can be simply or easily changed, as well as the above-described embodiments. In addition, the present disclosure will also include techniques that may be easily modified and implemented using the embodiments. Therefore, the scope of the present disclosure should not be limited to the above-described embodiments, and should be defined by the claims of the disclosure, which will be described later, and equivalents thereto.

## Claims

1. An electronic device (1000) comprising:
a reception circuit (1400) configured to receive input image data; and
a processor (1100) configured to perform at least one of:
a first operation of adjusting pixel values of first object pixels representing image data corresponding to noise data (ND1), among input pixels of the input image data,
a second operation of determining sequentially adjacent line pixels among the input pixels based on pixel values of the input pixels (PX1-PX7), and adjusting pixel values of second object pixels determined from among the input pixels based on the number of line pixels,
a third operation of adjusting coordinate values of the input pixels based on coordinate values of inflection pixels (CP1-CP14) determined based on rates of change in the coordinate values between the line pixels, and
a fourth operation of adjusting pixel values of the input pixels such that the input pixels having pixel values within a first range (PI1) have pixel values within a second range (PI2), a magnitude of the second range being greater than a magnitude of the first range.

2. The electronic device of claim 1, wherein the input image data is data on an X-ray image of a human body, and
wherein the electronic device further comprises an artificial intelligence system (1500) configured to be trained to obtain, from new input image data, a bone age information of the human body represented by the new input image data based on data obtained by performing at least one of the first operation to the fourth operation on the input image data.

3. The electronic device of claim 1 or claim 2, wherein the inflection pixels (CP1-CP14) are determined based on the rates of change and differences between the rates of change.

4. The electronic device of any one of claims 1 to 3, wherein the processor (1100) is configured to further perform a fifth operation of extracting region image data (IMP2) from the input image data based on an array of the line pixels.

5. The electronic device of any one of claims 1 to 4, wherein the processor (1100) is configured to further perform a sixth operation of obtaining object image data from the input image data based on a coordinate value of at least one of the inflection pixels (CP1-CP14) before the first to fourth operations, and
wherein the first to fourth operations are performed on the object image data, instead of the input image data.

6. The electronic device of any one of claims 1 to 5, wherein the processor (1100) is configured to determine the line pixels, based on whether each of differences between pixel values of the line pixels and pixel values of other pixels is equal to or greater than a threshold value, the pixel values of the other pixels being respectively adjacent to the line pixels.

7. The electronic device of any one of claims 1 to 6, wherein the line pixels comprise a first pixel (PG11, PG21) and a second pixel (PG21, PG31), and
wherein a first rate of change (K1, K2) between the first pixel and the second pixel, among the rates of change, is determined based on a difference between a coordinate value of the first pixel (y1, y2) and a coordinate value of the second pixel (y2, y3) on a first axis (Y), and based on a difference between a coordinate value of the first pixel (x1, x2) and a coordinate value of the second pixel (x2, x3) on a second axis (X) perpendicular to the first axis (Y).

8. The electronic device of claim 7, wherein the line pixels further comprise a third pixel (PG31, PG41), and
wherein if a difference between a second rate of change (K2, K3) between the second pixel and the third pixel and the first rate of change (K1, K2) is equal to or greater than a reference value, the second pixel (PG21, PG31) is included in the inflection pixels.

9. The electronic device of any one of claims 1 to 8, wherein the second object pixels are determined based on coordinate values of the line pixels.

10. The electronic device of any one of claims 1 to 9, wherein the second object pixels correspond to an image of a region divided by the line pixels, among the images displayed by the input image data.
